# EUROPEAN PATENT SPECIFICATION

(11) **EP 2 848 218 B1**
(45) Date of publication and mention of the grant of the patent: **04.01.2017**
(21) Application number: 14184406.8
(22) Date of filing: 11.09.2014
(51) Int. Cl.: A61B 17/3203, B26F 3/00, A61B 17/00

(54) **Liquid ejecting apparatus and medical device**
Flüssigkeitsausstoßende Vorrichtung und medizinische Vorrichtung
Appareil d'éjection de liquide et dispositif médical

(30) Priority: 11.09.2013 JP 2013188257
(43) Date of publication of application: 18.03.2015
(73) Proprietor: Seiko Epson Corporation, Shinjuku-ku Tokyo 163-0811 (JP)
(72) Inventor: Uchida, Kazuaki, Nagano 392-8502 (JP); Kojima, Hideki, Nagano 392-8502 (JP); Sekino, Hirokazu, Nagano 392-8502 (JP); Seto, Takeshi, Nagano 392-8502 (JP)
(74) Representative: Hoffmann Eitle

(56) References cited:
- GB-A- 2 495 248
- JP-A- 2010 053 767
- US-A1- 2010 082 053
- US-A1- 2011 037 795
- US-A1- 2012 176 431
- US-A1- 2013 218 184

## Description

### BACKGROUND

### 1. Technical Field

The present invention relates to ejection of a liquid.

### 2. Related Art

In a liquid ejecting apparatus used as a medical device, there is known a method of measuring an acceleration of an ejection port and selecting a mode of liquid ejection on the basis of the acceleration (see, for example, JP-A-2012-143374, upon which the preamble of claim 1 is based).

The above-mentioned technique of the related art is excellent in exhibiting incision or excision ability as intended by an operator by switching an ej ection mode according to the movement velocity of an ejection port. The inventors have found a method of suitably performing a change in the ability of incision or the like based on the movement velocity of a liquid ejecting mechanism by further improving this technique. Besides, a reduction in the size of, a reduction in the cost of, the resource saving of, the manufacturing facilitation of, an improvement in the usability of a device, and the like have been required. The inventors also have attempted a solution for such a problem.

### SUMMARY

An advantage of some aspects of the invention is to solve at least a part of the problems described above, and the invention can be implemented as the following forms.

The present invention is as defined in the appended claims.

According to claim 1, at least one of a voltage and a supplied flow rate (hereinafter, also referred to as a "supply flow rate") which are associated with an excision depth is changed depending on the movement velocity of an ejection port, and thus it is possible to adjust excision ability in accordance with the movement velocity of the ejection port. In addition, when the movement velocity of the liquid ejecting mechanism becomes faster, the voltage is changed so that the excision ability is improved, and thus it is possible to stabilize the excision depth.

According to claim 2, both the voltage and the liquid flow rate are changed depending on the movement velocity of the ejection port, and thus it is possible to adjust the excision ability in accordance with the movement velocity of the ejection port, and to realize flow rate control based on a change in the voltage.

According to claim 3, when the movement velocity becomes faster, the supply flow rate is increased, and thus it is possible to stabilize the excision depth. Further, according to this aspect, when the voltage becomes higher, the supply flow rate is increased. When the voltage becomes higher, a required flow rate is increased. Therefore, according to the aspect, when a change in the required flow rate occurs, there is an increasing possibility of an appropriate supply flow rate being secured.

According to claim 4, when the movement velocity becomes faster, the drive frequency is changed so that the excision ability is improved, and thus it is possible to stabilize the excision depth. Further, in the third velocity or higher, the drive frequency is able to be changed, whereas, when the voltage is not changed, the drive frequency can be determined with the exception of the influence of a change in voltage.

According to claim 5, it is possible to adjust the excision ability in accordance with the movement velocity of the ejection port, using three parameters.

The invention can be implemented in the form other than the medical device stated above.

### BRIEF DESCRIPTION OF THE DRAWINGS

The invention will be described with reference to the accompanying drawings, wherein like numbers reference like elements.
Fig. 1 is a configuration diagram illustrating a liquid ejecting apparatus.
Fig. 2 is an internal structure diagram illustrating the liquid ejecting mechanism.
Fig. 3 is a graph illustrating a drive waveform.
Fig. 4 is a flow diagram illustrating an ejection process (Embodiment 1).
Figs. 5A and 5B are graphs illustrating a relationship between each parameter and a movement velocity (Embodiment 1).
Fig. 6 is a graph illustrating a state where a drive waveform changes.
Fig. 7 is a graph illustrating a relationship between a supply flow rate and a required flow rate.
Fig. 8 is a graph illustrating a relationship between a required flow rate and a peak voltage.
Fig. 9 is a graph illustrating a relationship between a required flow rate and a drive frequency.
Fig. 10 is a graph illustrating a relationship between an ejection pressure and a peak voltage.
Fig. 11 is a graph illustrating a relationship between an excision depth and a peak voltage.
Fig. 12 is a flow diagram illustrating an ejection process (Embodiment 2).
Figs. 13A and 13B are graphs illustrating a relationship between each parameter and a movement velocity (Embodiment 3).

### DESCRIPTION OF EXEMPLARY EMBODIMENTS

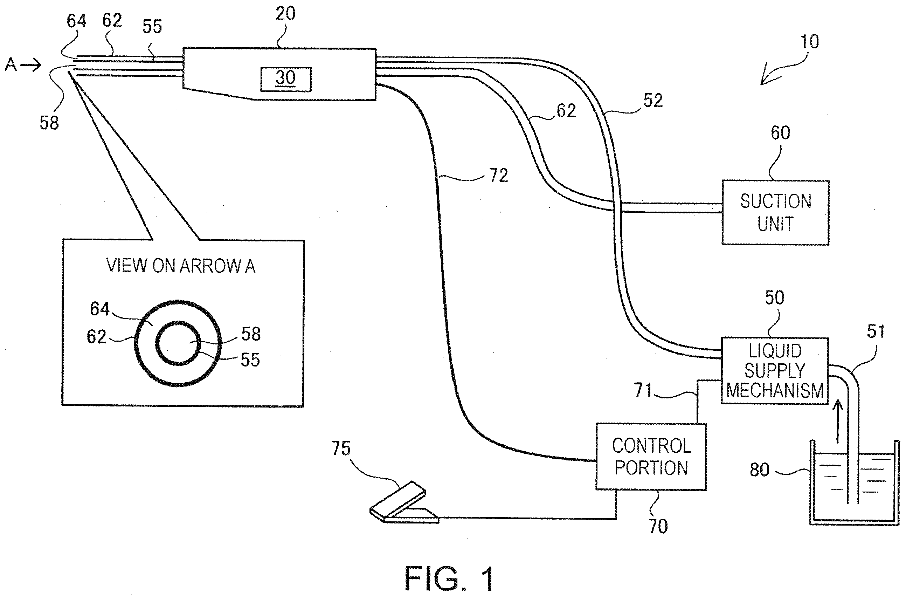

Embodiment 1 will be described below. Fig. 1 shows a configuration of a liquid ejecting apparatus 10. The liquid ejecting apparatus 10 is a medical device which is used in a medical institution, and has a function of incising or excising an affected part by ejecting a liquid onto the affected part.

The liquid ejecting apparatus 10 includes a liquid ejecting mechanism 20, a liquid supply mechanism 50, a suction unit 60, a control portion 70, and a liquid container 80. The liquid supply mechanism 50 and the liquid container 80 are connected to each other by a connection tube 51. The liquid supply mechanism 50 and the liquid ejecting mechanism 20 are connected to each other by a liquid supply channel 52. The connection tube 51 and the liquid supply channel 52 are formed of a resin. The connection tube 51 and the liquid supply channel 52 may be formed of materials (for example, metals) other than a resin.

The liquid container 80 reserves a physiological saline solution. Pure water or a drug solution may be reserved instead of the physiological saline solution. The liquid supply mechanism 50 supplies a liquid suctioned from the liquid container 80 through the connection tube 51 by the driving of a built-in pump to the liquid ejecting mechanism 20 through the liquid supply channel 52.

The liquid ejecting mechanism 20 is an appliance which is manipulated by a user of the liquid ejecting apparatus 10 with the mechanism held in his/her hand. A user incises or excises an affected part by applying a liquid, intermittently ejected from an ejection port 58, to the affected part.

The control portion 70 transmits a drive signal to a pulsation generation portion 30 built into the liquid ejecting mechanism 20 through a signal cable 72. The control portion 70 controls a flow rate of a liquid which is supplied to the pulsation generation portion 30 by controlling the liquid supply mechanism 50 through a control cable 71. A foot switch 75 is connected to the control portion 70. When a user turns on the foot switch 75, the control portion 70 controls the liquid supply mechanism 50 to execute the supply of a liquid to the pulsation generation portion 30, and transmits a drive signal to the pulsation generation portion 30 to generate pulsation in the pressure of the liquid supplied to the pulsation generation portion 30. The details of the mechanism of pulsation generation and the ejection control of a liquid from the liquid ejecting mechanism 20 will be described later.

The suction unit 60 is used for suctioning a liquid or an excised substance in the vicinity of the ejection port 58. The suction unit 60 and the liquid ejecting mechanism 20 are connected to each other by a suction channel 62. While a switch for bringing the suction unit 60 into operation is turned on, the suction unit 60 suctions the inside of the suction channel 62 at all times. The suction channel 62 passes through the inside of the liquid ejecting mechanism 20, and is opened in the vicinity of the apical end of an ejection tube 55.

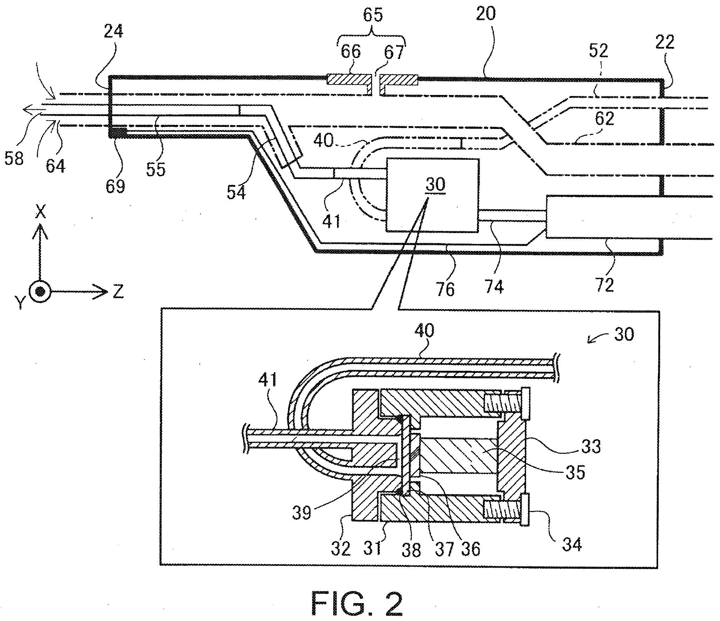

The suction channel 62 is covered with the ejection tube 55 extending from the apical end of the liquid ejecting mechanism 20. For this reason, as shown in a view taken in the direction of arrow A of Fig. 1, the wall of the ejection tube 55 and the wall of the suction channel 62 form a substantially concentric cylinder. A channel into which a suctioned substance suctioned from a suction port 64 which is the apical end of the suction channel 62 flows is formed between the outer wall of the ejection tube 55 and the inner wall of the suction channel 62. The suctioned substance is suctioned to the suction unit 60 through the suction channel 62. Meanwhile, this suction is adjusted by a suction adjustment mechanism 65 described later with reference to Fig. 2.

Fig. 2 shows an internal structure of the liquid ejecting mechanism 20. The liquid ejecting mechanism 20 has the pulsation generation portion 30, an inlet channel 40, an outlet channel 41, a connection tube 54, an acceleration sensor 69 built-in, and includes the suction force adjustment mechanism 65.

The pulsation generation portion 30 generates pulsation in the pressure of a liquid which is supplied from the liquid supply mechanism 50 through the liquid supply channel 52 to the liquid ejecting mechanism 20. The liquid having the pulsation of pressure generated therein is supplied to the ejection tube 55. The liquid supplied to the ejection tube 55 is intermittently ejected from the ejection port 58. The ejection tube 55 is formed of stainless steel. The ejection tube 55 may be formed of other metals such as brass, or other materials, such as reinforced plastic, which have a predetermined rigidity or higher.

As shown in the enlarged view of the lower portion of Fig. 2, the pulsation generation portion 30 includes a first case 31, a second case 32, a third case 33, a bolt 34, a piezoelectric element 35, a reinforcement plate 36, a diaphragm 37, a packing 38, an inlet channel 40 and an outlet channel 41. The first case 31 is a cylindrical member. The entirety of the first case 31 is hermetically sealed by the second case 32 being bonded to one end thereof, and the third case 33 being fixed to the other end thereof using the bolt 34. The piezoelectric element 35 is disposed in a space which is formed inside the first case 31.

The piezoelectric element 35 is a laminated piezoelectric element. One end of the piezoelectric element 35 is fastened to the diaphragm 37 with the reinforcement plate 36 interposed therebetween. The other end of the piezoelectric element 35 is fastened to the third case 33. The diaphragm 37 is created by a metal thin film. The peripheral edge of the diaphragm 37 is fastened to the first case 31, and is interposed between the first case 31 and the second case 32. A liquid chamber 39 is formed between the diaphragm 37 and the second case 32.

A drive signal is input from the control portion 70 through the signal cable 72 to the piezoelectric element 35. The signal cable 72 is inserted from a rear end 22 of the liquid ejecting mechanism 20. The signal cable 72 accommodates two electrode wires 74, and one signal wire 76 for an acceleration sensor. The electrode wire 74 is connected to the piezoelectric element 35 within the pulsation generation portion 30. The piezoelectric element 35 is expanded and contracted on the basis of the drive signal transmitted from the control portion 70. The volume of the liquid chamber 39 is fluctuated by the expansion and contraction of the piezoelectric element 35.

The inlet channel 40 into which a liquid flows is connected to the second case 32. The inlet channel 40 is bent in a U shape, and extends toward the rear end 22 of the liquid ejecting mechanism 20. The liquid supply channel 52 is connected to the inlet channel 40. The liquid supplied from the liquid supply mechanism 50 is supplied to the liquid chamber 39 through the liquid supply channel 52.

When the piezoelectric element 35 is expanded and contracted at a predetermined frequency, the diaphragm 37 vibrates. When the diaphragm 37 vibrates, the volume of the liquid chamber 39 is fluctuated, and the pressure of the liquid within the liquid chamber pulsates. A pressurized liquid flows out from the outlet channel 41 which is connected to the liquid chamber 39.

The ejection tube 55 is connected to the outlet channel 41 through the metal-made connection tube 54. The liquid flowing out to the outlet channel 41 is ejected from the ejection port 58 through the connection tube 54 and the ejection tube 55.

The suction force adjustment mechanism 65 is used for adjusting a force in order for the suction channel 62 to suction a liquid or the like from the suction port 64. The suction force adjustment mechanism 65 includes an operating portion 66 and a hole 67. The hole 67 is a through-hole for connecting the suction channel 62 and the operating portion 66. When a user opens and closes the hole 67 with a finger of a hand grasping the liquid ejecting mechanism 20, the amount of air flowing into the suction channel 62 through the hole 67 is adjusted depending on the degree of the opening and closing, and thus the suction force of the suction port 64 is adjusted. The adjustment of the suction force may be realized by control using the suction unit 60.

The liquid ejecting mechanism 20 includes the acceleration sensor 69. The acceleration sensor 69 is a piezo-resistive 3-axis acceleration sensor. The 3 axes are respective axes of XYZ shown in Fig. 2. The X axis is in parallel with the passing-through direction of the hole 67, and an upward direction is a positive direction. The Z axis is in parallel with the long-axis direction of the ejection tube 55, and a direction in which a liquid is ejected is set to a negative direction. The Y axis is defined by a right-handed system with reference to the X axis and the Z axis.

As shown in Fig. 2, the acceleration sensor 69 is disposed in the vicinity of an apical end 24 of the liquid ejecting mechanism 20. A measurement result is input to the control portion 70 through the signal wire 76 for an acceleration sensor.

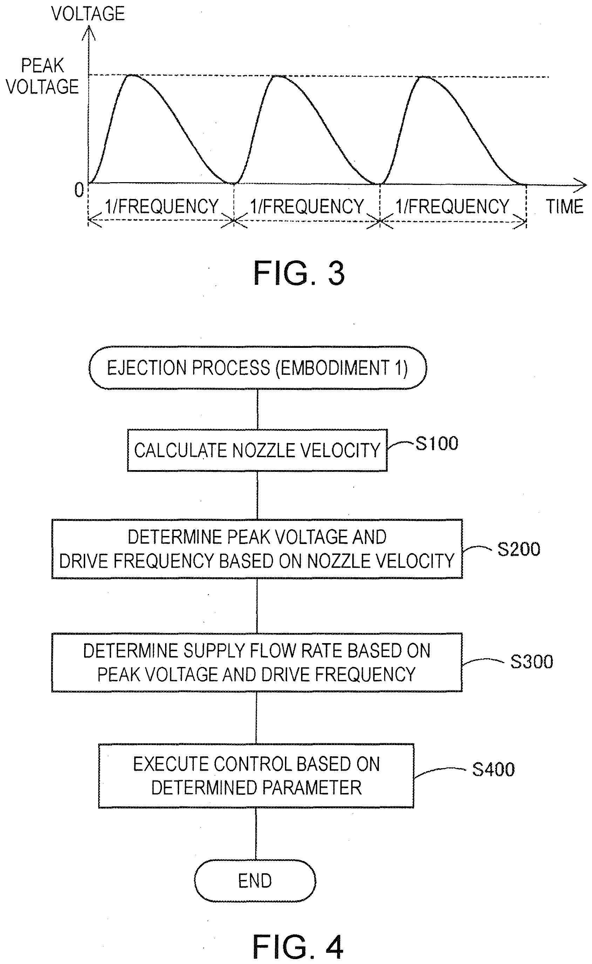

Fig. 3 is a graph illustrating a waveform of a drive signal (hereinafter, referred to as a "drive waveform") which is input to the piezoelectric element 35. The vertical axis represents a voltage, and the horizontal axis represents a time. The drive waveform is described by a combination of sine curves. The peak voltage and frequency of the drive waveform is changed by an ejection process (described later along with Fig. 4).

The piezoelectric element 35 is deformed so that the volume of the liquid chamber 39 is contracted when the voltage value of a drive signal increases. This contraction is repeatedly generated by the drive signal being repeatedly input. As a result, a liquid is intermittently ejected.

Fig. 4 is a flow diagram illustrating an ejection process. The ejection process is repeatedly executed by the control portion 70 while the foot switch 75 is stepped on. Initially, a velocity S of the ejection port 58 is calculated (step S100). The term "velocity S" as used herein refers to an absolute value of velocity in the XY plane. That is, it is an absolute value of a velocity ignoring a velocity in a Z-axis direction. The velocity S is calculated on the basis of the 3-axis acceleration which is measured by the acceleration sensor 69.

The velocity S is calculated as a parameter influencing the excision depth of the affected part. This is because the excision ability acting on each local region of the affected part per unit time is influenced by a movement velocity between the ejection port 58 and the affected part. In the present embodiment, on the assumption that the affected part remains stationary, the velocity S is handled as the movement velocity between the affected part and the ejection port 58. Meanwhile, considering that the affected part moves due to respiration or the like, the velocity S may be handled as a relative velocity between the ejection port 58 and the affected part.

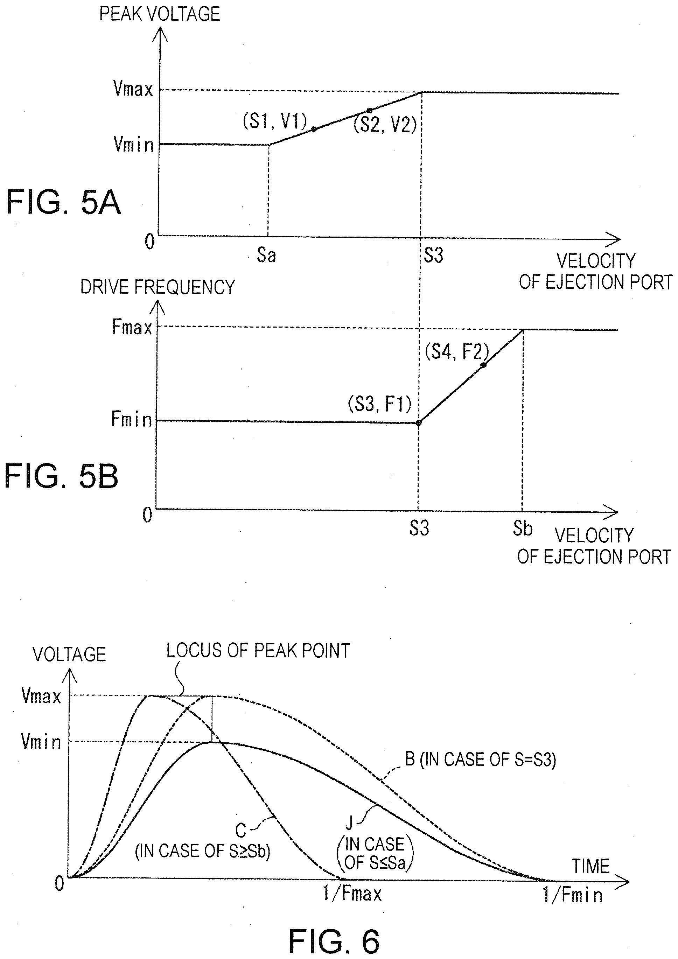

Subsequently, a peak voltage and a drive frequency are determined on the basis of the calculated velocity S (step S200). Figs. 5A and 5B are graphs illustrating a relationship between a peak voltage and a drive frequency, and the velocity S. Fig. 5A shows a peak voltage, and Fig. 5B shows a drive frequency, in the respective vertical axes. The horizontal axis is common with respect to the velocity S, and scales are coincident with each other in Figs. 5A and 5B.

As shown in Figs. 5A and 5B, in each velocity range of Sa ≤ velocity S ≤ S3 and S3 ≤ velocity S ≤ Sb, parameters having changing values are different from each other. That is, Sa, S3 and Sb are velocities which are previously determined as thresholds for switching changing parameters.

When the relation of velocity S ≤ Sa is satisfied, the peak voltage is fixed to Vmin which is a minimum value, and the drive frequency is fixed to Fmin which is a minimum value. When the parameters are set in this manner, the excision ability becomes lowest.

When the relation of Sa ≤ velocity S≤S3 is satisfied, the drive frequency is fixed to Fmin, whereas the peak voltage linearly increases with an increase in the velocity S. when the relation of velocity S = S3 is satisfied, the peak voltage is set to Vmax which is a maximum value. Vmin is set so that the excision ability does not decrease excessively. Vmax is set so that the load of the piezoelectric element 35 does not increase excessively.

When the relation of S3 ≤ velocity S ≤ Sb is satisfied, the peak voltage is fixed to Vmax, whereas the drive frequency linearly increases with an increase in the velocity S. When the relation of velocity S = Sb is satisfied, the drive frequency is set to Fmax which is a maximum value. Fmin is set to so that the excision ability does not decrease excessively and the intermittent ejection is realized. Fmax is set so that the load of the piezoelectric element 35 does not increase excessively. When the peak voltage and the drive frequency are changed in this manner, the drive waveform is changed.

Fig. 6 is a graph illustrating a state where the drive waveform is changed. The vertical axis represents a voltage, and the horizontal axis represents a time. Fig. 6 illustrates three drive waveforms. A curve J represents a drive waveform when the peak voltage is set to Vmin and the drive frequency is set to Fmin. That is, the curve J represents a drive waveform when the above-mentioned relation of velocity S ≤ Sa is satisfied. A curve B represents a drive waveform when the peak voltage is set to Vmax and the drive frequency is set to Fmin. That is, the curve B represents a drive waveform when the above-mentioned relation of velocity S = S3 is satisfied. A curve C represents a drive waveform when the peak voltage is set to Vmax and the drive frequency is set to Fmax. That is, the curve C represents a drive waveform when the above-mentioned relation of velocity S ≥ Sb is satisfied.

When the peak voltage becomes higher, the amount of expansion and contraction of the piezoelectric element 35 increases. Therefore, a fluctuation ratio between volume fluctuations of the liquid chamber 39 becomes higher. The term "fluctuation ratio" as used herein refers to a value obtained by dividing a maximum volume in the volume fluctuations by a minimum volume. When a ratio between volume fluctuations becomes higher, pressure fluctuation within the liquid chamber 39 increases. When the pressure fluctuation within the liquid chamber 39 increases, the liquid is ejected with great force. Further, when the peak voltage becomes higher, the amount of the liquid ejected increases. When the peak voltage becomes higher due to these actions, the excision ability increase. As a result, even when the excision ability acting per unit area by the velocity S becoming faster decreases, the decrease is offset, and the excision depth is stabilized. Meanwhile, the term "offset" as used herein is not limited to a case where the excision depth does not change entirely even when the velocity S changes, and include a case where at least a portion of an influence due to the velocity S changing is diminished.

When the drive frequency becomes higher, the number of times at which the liquid is ejected per unit time increases. Further, in a case of the present embodiment, as shown in Fig. 6, when the drive frequency becomes higher, a rise time is shortened. The term "rise time" as used herein refers to a time taken for the voltage value of the drive signal to reach a peak from zero. When the rise time is shortened, the contraction of the liquid chamber 39 is executed in a short time. As a result, the liquid is ejected with great force. When the drive frequency becomes higher due to these actions, the excision ability increases, and the excision depth is stabilized even when the velocity S becomes faster.

After the peak voltage and the drive frequency are determined as described above, a supply flow rate is determined (step S300), and control is executed on the basis of the peak voltage, the drive frequency and the supply flow rate which are determined (step S400). The term "supply flow rate" as used herein refers to a volumetric flow rate of the liquid which is supplied by the liquid supply mechanism 50.

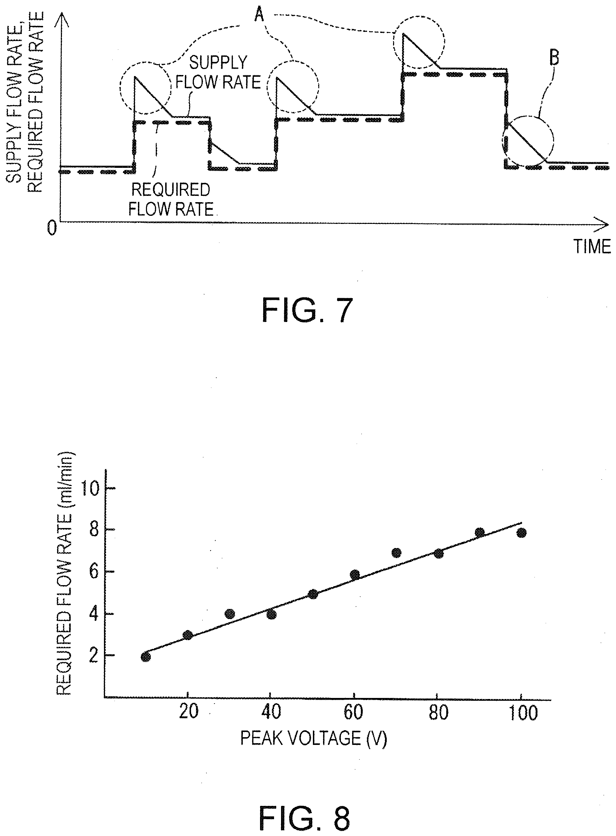

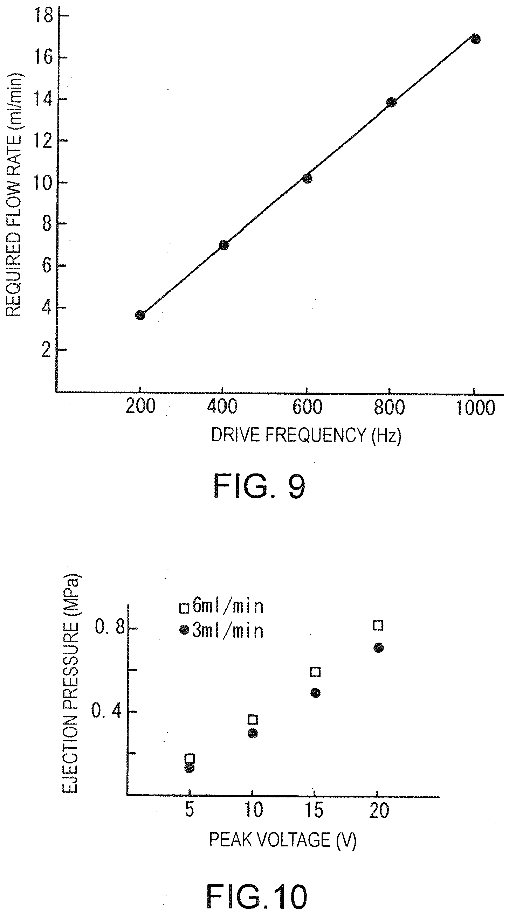

Fig. 7 is a graph conceptually illustrating a method of determining a supply flow rate. The vertical axis represents a supply flow rate and a required flow rate, and the horizontal axis represents a time. The required flow rate refers to a required flow rate in order for the liquid chamber 39 to be filled with a liquid, and the calculation method thereof will be described later along with Figs. 8 and 9.

The supply flow rate is set to a value which slightly exceeds the required flow rate in principle. When the supply flow rate falls below the required flow rate, ejection may not possibly be executed even in a case where the volume of the liquid chamber 39 is contracted. When the ejection is not normally executed in this manner, the excision ability may decrease. On the other hand, when the supply flow rate drastically exceeds the required flow rate, a liquid is ejected even at a timing when the ejection is interrupted in order to realize the intermittent ejection, the intermittent ejection may not be able to be normally executed. Further, when the supply flow rate drastically exceeds the required flow rate, the affected part is filled with a liquid, which may cause interference with a surgical operation. Thus, as described above, it is preferable that the supply flow rate be a value which slightly exceeds the required flow rate.

In the present embodiment, when the required flow rate changes, the supply flow rate is temporarily increased. When the required flow rate is Fd, and the required flow rate becomes 2xFd from a state where the supply flow rate is Fs (>Fd), the supply flow rate is temporarily set to 3xFs, and then is made to gradually converge on 2xFs. Places expressed as A in a curve showing the supply flow rate of Fig. 7 conceptually shows this flow rate control.

Alternatively, when the required flow rate is Fd, and the required flow rate becomes 0.5xFd from a state where the supply flow rate is Fs, the supply flow rate is temporarily set to 0. 75xFs, and then is made to gradually converge on 0.5×Fs. A place expressed as B in the curve showing the supply flow rate of Fig. 7 conceptually shows this flow rate control.

In this manner, when the required flow rate changes, the supply flow rate is made to be larger than a target value temporarily, and thus the ejection of the liquid is prevented from not being able to be normally executed with the lack of the supply flow rate due to control delay or undershoot.

Fig. 8 is a graph illustrating experimental results regarding a relationship between the required flow rate and the peak voltage. Each point on the graph represents experimental results, and the straight line represents an approximation straight line of each point. As shown in Fig. 8, when the peak voltage is increased two times, the required flow rate is increased approximately 1.5 times.

Fig. 9 is a graph illustrating experimental results regarding a relationship between the required flow rate and the drive frequency. Each point on the graph represents experimental results, and the straight line represents an approximation straight line of each point. As shown in Fig. 9, when the drive frequency is increased two times, the required flow rate is increased approximately two times.

The determination of the supply flow rate in step S300 shown in Fig. 4 is realized by calculating the required flow rate on the basis of the relationships shown in Figs. 8 and 9 and calculating the supply flow rate on the basis of the calculated required flow rate.

As described above, the supply flow rate is determined on the basis of the relationship with the required flow rate, and also influences the excision ability. Fig. 10 is a graph illustrating a relationship between the ejection pressure and the peak voltage when the supply flow rate is classified into two cases. The drive frequency is set to the same value in any case. Even when the supply flow rate is set to any case of 3 ml/min and 6 ml/min, the ejection pressure increases with an increase in the peak voltage. This shows an improvement in the excision ability with an increase in the peak voltage described above.

As shown in Fig. 10, in each peak voltage, the case of 6 ml/min has the ejection pressure higher than the case of 3 ml/min.

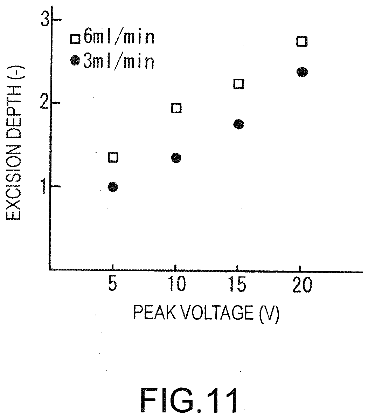

Fig. 11 is a graph illustrating a relationship between the excision depth and the peak voltage when the supply flow rate is classified into two cases. This excision depth is shown by a value which is made non-dimensional by setting a case where the peak voltage is 5V and the supply flow rate is 3 ml/min to 1. The drive frequency is set to the same value in any case.

Similarly to a case of the ejection pressure (Fig. 10), even when the supply flow rate is set to any case of 3 ml/min and 6 ml/min, the excision depth increases with an increase in the peak voltage, the case of 6 ml/min has the excision depth larger than the case of 3 ml/min in each peak voltage.

In any of the graphs shown in Figs. 10 and 11, an increase in the supply flow rate shows contribution to an improvement in the excision ability. The relationship between velocity S and the peak voltage and the relationship between the velocity S and the drive frequency described above are determined with the addition of a change in the excision ability by the supply flow rate which is determined on the basis of the relationship with the required flow rate.

As described above, as the velocity S increases, the peak voltage is changed so that the excision ability is improved, thereby allowing the excision depth to be stabilized. Further, when the peak voltage reaches a maximum value, the drive frequency and the peak voltage are changed, thereby allowing the excision depth to be stabilized.

According to the present embodiment, the range of the velocity S which changes the peak voltage and the drive frequency is separated, it is easy to determine values of the peak voltage and the drive frequency in each velocity range. Meanwhile, since the range of the velocity S which changes the drive frequency and the peak voltage is separated, the peak point of the drive waveform draws a locus having such a shape that a Γ shape is clockwise rotated by 90 degrees, as shown in Fig. 6, during a change in the drive waveform.

As an example, S1 to S4 shown in Figs. 5A and 5B are first to fourth velocities in the accompanying claims, V1 and V2 are first and second voltages, and F1 and F2 are first and second frequencies. The piezoelectric element 35 and the diaphragm 37 in the embodiment are an example of a volume fluctuation portion in the accompanying claims.

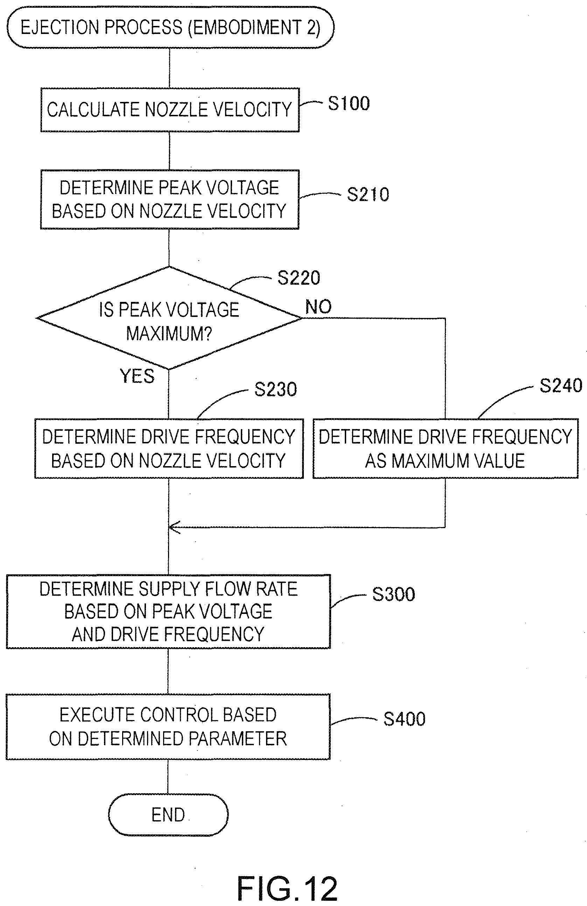

Embodiment 2 will be described below. In Embodiment 2, an ejection process shown in Fig. 12 is executed instead of the ejection process shown in Fig. 4. A hardware configuration is the same as in Embodiment 1, and thus the description thereof will be omitted. Step S100, step S300 and step S400 in the ejection process of Embodiment 2 are the same as in Embodiment 1, and thus the description thereof will be omitted. In Embodiment 2, step S210 to step S240 are executed instead of step S200 in Embodiment 1.

After the velocity S is calculated (step S100), the peak voltage is determined on the basis of the calculated velocity S (step S210). A method of determining the peak voltage is the same as in Embodiment 1. That is, a case of velocity S ≤ Sa is fixed to Vmin, a case of Sa ≤ velocity S ≤ Sb linearly increases, and a case of Sb ≤ velocity S is fixed to Vmax.

Next, it is determined whether the peak voltage is set to a maximum value (Vmax) (step S220). When the peak voltage is set to a value less than the maximum value (step S220, NO), the drive frequency is set to a minimum value (Fmin) (step S240). Setting of the peak voltage to a value less than the maximum value means ability to improve the excision ability due to a change in the peak voltage. Thus, since it is not necessary to improve the excision ability by changing the value of the drive frequency, the drive frequency is set to the minimum value.

On the other hand, when the peak voltage is set to the maximum value (step S220, YES), the drive frequency is determined on the basis of the velocity S (step S230). Setting of the peak voltage to the maximum value means inability to improve the excision ability due to a change in the peak voltage. Consequently, step S230 is executed in order to improve the excision ability by changing the value of the drive frequency. In Embodiment 2, it is also possible to obtain the same control result as in Embodiment 1.

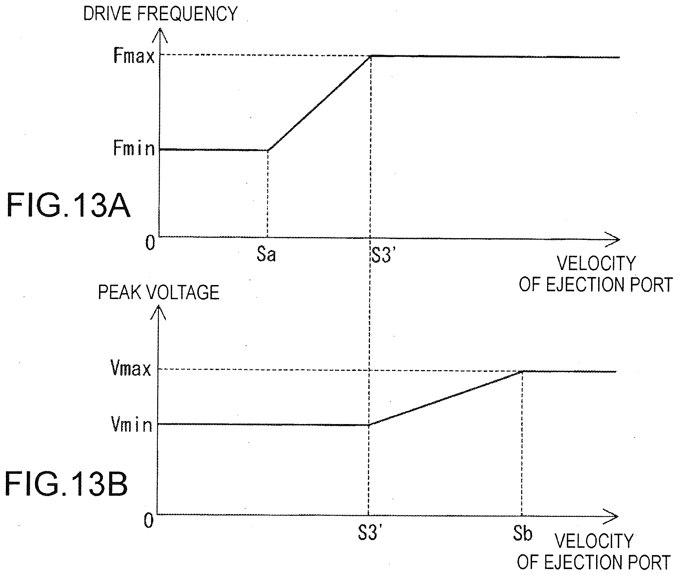

Embodiment 3 will be described below. In Embodiment 3, step S200 of the ejection process is executed on the basis of the relationships shown in Figs. 13A and 13B instead of the relationship between the peak voltage and the drive frequency, and the velocity S in Embodiment 1 shown in Figs. 5A and 5B. Fig. 13A shows a drive frequency, and Fig. 13B shows a peak voltage, in the respective vertical axes. The horizontal axis is common with the velocity S, and scales are coincident with each other in Figs. 13A and 13B.

As shown in Figs. 13A and 13B, the drive frequency increases in the velocity range of Sa≤ velocity S ≤ S3', and the peak voltage increases in the velocity range of S3' ≤ velocity S ≤ Sb. That is, unlike Embodiment 1, the excision ability is first improved by a change in the drive frequency, and the excision ability is improved by a change in the peak voltage when the drive frequency reaches the maximum value.

In Sa and Sb of Embodiment 3, the same values as the values adopted in Embodiment 1 are adopted. Sa is a velocity in which an improvement in the excision ability is preferably started, and which is because it is common with Embodiment 1 in this viewpoint. Sb is the slowest velocity of velocities in which the drive frequency and the peak voltage are set to the maximum value, and thus is set to the same value as in Embodiment 1 even when a change order is counterchanged. S3' is a value which is set as a velocity when the drive frequency reaches the maximum value, and thus a value different from S3 in Embodiment 1 is adopted. Sa and Sb may be, of course, values different from those in Embodiment 1, and S3' may be the same value as S3.

In Embodiment 3, it is also possible to stabilize the excision depth similarly to Embodiment 1. Regarding whether any of the peak voltage and the drive frequency is preferentially changed, it is considered that one of the peak voltage and the drive frequency in which the excision depth is further stabilized is selected on the basis of the characteristics of the piezoelectric element 35.

Embodiment 4 will be described below. In a liquid ejection method, laser light may be used. In the ejection method using laser light, for example, pressure fluctuation occurring by intermittently irradiating a liquid with laser light and vaporizing the liquid may be used.

A liquid ejecting apparatus in Embodiment 4 includes an output portion that outputs laser light into a liquid chamber in accordance with a drive signal, and an ejection port that ejects a liquid from the liquid chamber, and a control portion that outputs laser light to the output portion by a first output when a movement velocity of the ejection port is a first velocity and outputs laser light to the output portion by a second output higher than the first output when the movement velocity is a second velocity faster than the first velocity. According to such an aspect, energy of laser light in one-time emission increases, and pressure fluctuation within the liquid chamber increases. As a result, a liquid is ejected with great force to thereby increase the excision ability, and the excision depth is stabilized even when the movement velocity becomes faster.

In addition, the control portion may set a maximum voltage of the drive signal to a first voltage when the movement velocity is the first velocity, and may set the maximum voltage of the drive signal to a second voltage higher than the first voltage when the movement velocity is the second velocity. In such an aspect, it is possible to easily control an output of laser light. Energy of laser light per one-time output easily rises by adjusting a voltage, and the excision depth is stabilized even when the movement velocity becomes faster.

In Embodiment 4, the control portion may change a frequency of the drive signal in accordance with the movement velocity. In such an aspect, the output of laser light can be adjusted by methods other than a change in the maximum voltage, and the excision depth is stabilized even when the movement velocity becomes faster through an easy operation.

Further, the control portion may set a frequency of the drive signal to a first frequency when the maximum voltage is the first and second voltages, and may set the frequency of the drive signal to a second frequency higher than the first frequency when the maximum voltage is a third voltage higher than the second voltage. In such an aspect, when the maximum voltage is the first and second voltages, the output is controlled by a change in the maximum voltage without changing the frequency of the drive signal, and thus the values of the first and second voltages are easily determined.

In Embodiment 4, the control portion may set the maximum voltage to a third voltage and set a frequency of the drive signal to the second frequency when the movement velocity is the third velocity faster than the second velocity, and may set the maximum voltage to the third voltage and set the frequency of the drive signal to a third frequency higher than the second frequency when the movement velocity is a fourth velocity faster than the third velocity. In such an aspect, when the movement velocity is the third and fourth velocities, the output is controlled by a change in the frequency without changing the maximum voltage of the drive signal, and thus the values of the second and third frequency are easily determined.

In addition, a liquid supply portion that supplies a liquid to the liquid chamber at a flow rate which is set by the control portion is included. The control portion may set the flow rate to a first flow rate when the movement velocity is the first velocity, and may set the flow rate to a second flow rate higher than the first flow rate when the movement velocity is the second velocity. In such an aspect, the supplied flow rate can be appropriately set.

Further, the control portion may change the maximum voltage and the frequency of the drive signal in accordance with the movement velocity. According to the aspect, the output of laser light can be changed by the maximum voltage and the frequency of the drive signal.

The invention is not limited to the aforementioned embodiments, examples, and modification examples of this specification, and can be implemented by various configurations without the gist of the invention. For example, technical features in the embodiments, examples, and modification examples which correspond to the technical features in the respective aspects described in the summary of the invention can be appropriately replaced or combined in order to solve some or all of the aforementioned problems, or to achieve some or all of the aforementioned effects. The technical features can be appropriately deleted as long as they are not described as essential features in this specification. For example, the following is exemplified.

The drive frequency may not be changed. That is, the adjustment of the excision ability may be realized by changing the peak voltage and the supply flow rate.

The adjustment of the excision ability may be realized by only changing the peak voltage without changing the drive frequency and the supply flow rate.

Alternatively, the adjustment of the excision ability may be realized by only changing the supply flow rate without changing the peak voltage and the drive frequency. When a configuration is adopted in which the peak voltage and the drive frequency are not changed, it is possible to simplify the configuration of the control portion.

The peak voltage, the drive frequency and the supply flow rate may be determined using a function.

A velocity range in which the peak voltage is fluctuated and the velocity range in which the drive frequency is fluctuated may overlap each other.

The drive waveform may not be a combination of sine curves, and may be increased or decreased, for example, in a stepwise manner.

A relationship between each of the peak voltage and the drive frequency and the velocity of the ejection port may be specified in a curve manner, and may be specified in a stepwise manner.

The drive frequency may be changed in a state where a rise time is fixed. That is, the drive frequency may be changed by changing a time until the voltage of the drive signal reaches zero from a peak. In this manner, when the drive frequency is determined with respect to the movement velocity, the influence of a change in the rise time can be excluded, and thus the determination of the drive frequency is facilitated.

The velocity of the ejection port may be calculated, for example, by the acceleration sensor which is installed on the apical end of the ejection port. In this case, calculation results are considered to be more accurate.

Alternatively, the velocity of the ejection port may be calculated using image processing. For example, the velocity of the ejection port may be calculated by installing a marker on the apical end of the ejection port, and grasping the movement of the marker using a camera.

When a robot operates the liquid ejecting apparatus, the velocity of the ejection port can be grasped by the robot, and thus the grasped value may be used without requiring calculation.

The movement velocity of the ejection port may be calculated with the addition of the movement velocity of the affected part. The measurement of the movement velocity of the affected part may be realized by predicting or measuring movement due to respiration or pulsation. Meanwhile, the detection of the movement velocity may be performed at a place moving in association with the movement of the ejection port without being limited to that of the ejection port, and the movement velocity of the liquid ejecting mechanism may be detected.

In addition, control for ejecting a liquid may be performed so that at least one of a predetermined liquid amount, energy of a predetermined liquid, a predetermined pressure of a liquid, and the like is given to an object to which a liquid is ejected regardless of a change in the movement velocity of the ejection port, and control may be performed in which two or more physical quantities of a predetermined liquid amount, energy of a predetermined liquid, and a predetermined pressure of a liquid may be combined.

The type of the acceleration sensor may be a capacitance type and may be a heat detection type. In addition, a sensor may be used which is capable of detecting the movement velocity of the ejection port indirectly or directly without being limited to acceleration.

In a liquid ejection method, laser light may be used. In the ej ection method using laser light, for example, pressure fluctuation occurring by intermittently irradiating a liquid with laser light and vaporizing the liquid may be used.

## Claims

1. A medical device having a liquid ejecting apparatus (10) comprising:
a liquid ejecting mechanism (20) which is provided with a liquid chamber (39) and a volume fluctuation portion (35) that is configured to fluctuate a volume within the liquid chamber (39);
a liquid supply portion (50) configured to supply a liquid to the liquid chamber; and
a control portion (70) configured to control the volume fluctuation portion (35) and the liquid supply portion (50),
wherein the control portion (70) is configured to change at least one of a voltage applied to the volume fluctuation portion (35) and a flow rate of a liquid supplied to the liquid chamber (39), in accordance with a movement velocity of the liquid ejecting mechanism (20),
**characterized in that** the control portion (70) is configured to set the voltage to a first voltage when the movement velocity of the liquid ejecting mechanism (20) is a first velocity, and set the voltage to a second voltage higher than the first voltage when the movement velocity is a second velocity faster than the first velocity.

2. The medical device according to claim 1, wherein the control portion (70) is configured to change the voltage and the flow rate.

3. The medical device according to claim 1 or 2, wherein the control portion (70) is configured to set the flow rate of the liquid supplied to the liquid chamber (39) to a first flow rate when the movement velocity is the first velocity, and set the flow rate of the liquid supplied to the liquid chamber (39) to a second flow rate higher than the first flow rate when the movement velocity is the second velocity.

4. The medical device according to claim 3, wherein a drive signal is applied to the volume fluctuation portion (35), and
the control portion (70) is configured to change the voltage when the movement velocity is equal to or less than a third velocity faster than the second velocity, set a frequency of the drive signal to a first frequency when the movement velocity is the third velocity, and set the frequency of the drive signal to a second frequency higher than the first frequency when movement velocity is a fourth velocity faster than the third velocity.

5. The medical device according to any one of the preceding claims, wherein the control portion (70) is configured to control the voltage, the flow rate, and the frequency of the drive signal in accordance with the movement velocity.

## Patentansprüche

1. Medizinische Vorrichtung mit einer Flüssigkeitsausstoßeinrichtung (10), umfassend:
einen Flüssigkeitsausstoßmechanismus (20), der mit einer Flüssigkeitskammer (39) und einem Volumenfluktuationsabschnitt (35), der dazu ausgestaltet ist, ein Volumen innerhalb der Flüssigkeitskammer (39) zu fluktuieren, versehen ist;
einen Flüssigkeitszuführungsabschnitt (50), der dazu ausgestaltet ist, der Flüssigkeitskammer (39) eine Flüssigkeit zuzuführen; und
einen Steuerungsabschnitt (70), der dazu ausgestaltet ist, den Volumenfluktuationsabschnitt (35) und den Flüssigkeitszuführungsabschnitt (50) zu steuern,
wobei der Steuerungsabschnitt (70) dazu ausgestaltet ist, eine an den Volumenfluktuationsabschnitt (35) angelegte Spannung und/oder Strömungsrate einer der Flüssigkeitskammer (39) zugeführten Flüssigkeit in Übereinstimmung mit einer Bewegungsgeschwindigkeit des Flüssigkeitsausstoßmechanismus (20) zu verändern,
**dadurch gekennzeichnet, dass** der Steuerungsabschnitt (70) dazu ausgestaltet ist, die Spannung auf eine erste Spannung einzustellen, wenn die Bewegungsgeschwindigkeit des Flüssigkeitsausstoßmechanismus (20) eine erste Geschwindigkeit ist, und die Spannung auf eine zweite Spannung, die höher als die erste Spannung ist, einzustellen, wenn die Bewegungsgeschwindigkeit eine zweite Geschwindigkeit ist, die höher als die erste Geschwindigkeit ist.

2. Medizinische Vorrichtung nach Anspruch 1, wobei der Steuerungsabschnitt (70) dazu ausgestaltet ist, die Spannung und die Strömungsrate zu verändern.

3. Medizinische Vorrichtung nach Anspruch 1 oder 2, wobei der Steuerungsabschnitt (70) dazu ausgestaltet ist, die Strömungsrate der Flüssigkeit, die der Flüssigkeitskammer (39) zugeführt wird, auf eine erste Strömungsrate einzustellen, wenn die Bewegungsgeschwindigkeit die erste Geschwindigkeit ist, und die Strömungsrate der Flüssigkeit, die der Flüssigkeitskammer (39) zugeführt wird, auf eine zweite Strömungsrate höher als die erste Strömungsrate einzustellen, wenn die Bewegungsgeschwindigkeit die zweite Geschwindigkeit ist.

4. Medizinische Vorrichtung nach Anspruch 3, wobei ein Treibersignal an den Volumenfluktuationsabschnitt (35) angelegt wird und
der Steuerungsabschnitt (70) dazu ausgestaltet ist, die Spannung zu ändern, wenn die Bewegungsgeschwindigkeit gleich oder niedriger als eine dritte Geschwindigkeit ist, die höher als die zweite Geschwindigkeit ist, eine Frequenz des Treibersignals auf eine erste Frequenz zu setzen, wenn die Bewegungsgeschwindigkeit die dritte Geschwindigkeit ist, und die Frequenz des Treibersignals auf eine zweite Frequenz zu setzen, die höher als die erste Frequenz ist, wenn die Bewegungsgeschwindigkeit eine vierte Geschwindigkeit höher als die dritte Geschwindigkeit ist.

5. Medizinische Vorrichtung nach einem der vorhergehenden Ansprüche, wobei der Steuerungsabschnitt (70) dazu ausgestaltet ist, die Spannung, die Strömungsrate und die Frequenz des Treibersignals in Übereinstimmung mit der Bewegungsgeschwindigkeit zu steuern.

## Revendications

1. Dispositif médical ayant un appareil d'éjection de liquide (10) comprenant :
un mécanisme d'éjection de liquide (20) qui est doté d'une chambre de liquide (39) et d'une portion de fluctuation de volume (35) qui est configurée pour faire fluctuer un volume à l'intérieur de la chambre de liquide (39) ;
une portion d'alimentation de liquide (50) configurée pour alimenter un liquide vers la chambre de liquide; et
une portion de commande (70) configurée pour commander la portion de fluctuation de volume (35) et la portion d'alimentation de liquide (50),
dans lequel la portion de commande (70) est configurée pour changer au moins un parmi une tension appliquée à la portion de fluctuation de volume (35) et un débit d'un liquide alimenté vers la chambre de liquide (39), conformément à une vitesse de mouvement du mécanisme d'éjection de liquide (20),
**caractérisé en ce que** la portion de commande (70) est configurée pour régler la tension à une première tension lorsque la vitesse de mouvement du mécanisme d'éjection de liquide (20) est une première vitesse, et régler la tension à une deuxième tension supérieure à la première tension lorsque la vitesse de mouvement est une deuxième vitesse supérieure à la première vitesse.

2. Dispositif médical selon la revendication 1, dans lequel la portion de commande (70) est configurée pour changer la tension et le débit.

3. Dispositif médical selon la revendication 1 ou 2, dans lequel la portion de commande (70) est configurée pour régler le débit du liquide alimenté vers la chambre de liquide (39) à un premier débit lorsque la vitesse de mouvement est la première vitesse, et régler le débit du liquide alimenté vers la chambre de liquide (39) à un deuxième débit supérieur au premier débit lorsque la vitesse de mouvement est la deuxième vitesse.

4. Dispositif médical selon la revendication 3, dans lequel un signal d'attaque est appliqué à la portion de fluctuation de volume (35), et
la portion de commande (70) est configurée pour changer la tension lorsque la vitesse de mouvement est égale ou inférieure à une troisième vitesse supérieure à la deuxième vitesse, régler une fréquence du signal d'attaque à une première fréquence lorsque la vitesse de mouvement est la troisième vitesse, et régler la fréquence du signal d'attaque à une deuxième fréquence supérieure à la première fréquence lorsque la vitesse de mouvement est une quatrième vitesse supérieure à la troisième vitesse.

5. Dispositif médical selon l'une quelconque des revendications précédentes, dans lequel la portion de commande (70) est configurée pour commander la tension, le débit et la fréquence du signal d'attaque conformément à la vitesse de mouvement.
